(19) Europäisches Patentamt
European Patent Office
Office européen des brevets

(11) Veröffentlichungsnummer: **0 003 343**
**B1**

# (12) EUROPÄISCHE PATENTSCHRIFT

(45) Veröffentlichungstag der Patentschrift: **07.01.81**

(51) Int. Cl.³: **C 07 C 49/12,** C 07 C 45/29

(21) Anmeldenummer: **79100181.1**

(22) Anmeldetag: **22.01.79**

## (54) Verfahren zur Herstellung von Glyoxal.

(30) Priorität: **26.01.78 DE 2803318**

(43) Veröffentlichungstag der Anmeldung: **08.08.79 Patentblatt 79/16**

(45) Bekanntmachung des Hinweises auf die Patenterteilung: **07.01.81 Patentblatt 81/1**

(84) Benannte Vertragsstaaten: **BE CH DE FR GB NL**

(56) Entgegenhaltungen:
**US-A-2 051 266**
**CHEMICAL ABSTRACTS, Vol. 61, Nr. 2, 20. Juli 1964, Columbus, Ohio, USA, N.V. KUZNETSOV et al.: "Synthesis of glyoxal and glycolic acid", Seite 1751 h**
**CHEMICAL ABSTRACTS, Vol. 62, Nr. 1, 4. Januar 1965, Columbus, Ohio, USA, M.S. BRODSKII et al.: "Preparation of glyoxal", Spalte 447**

(73) Patentinhaber: **BASF Aktiengesellschaft, Carl-Bosch-Strasse 38, D-6700 Ludwigshafen (DE)**

(72) Erfinder: **Diem, Hans, Dr., Feldbergstrasse 63, D-6800 Mannheim 25 (DE)**
Erfinder: **Dudeck, Christian, Dr., Odenwaldring 20, D-6703 Limburgerhof (DE)**
Erfinder: **Lehmann, Gunter, Bayernstrasse 58, D-6700 Ludwigshafen (DE)**
Erfinder: **Matthias, Guenther, Dr., Meergartenweg 25 a, D-6710 Frankenthal (DE)**
Erfinder: **Petri, Norbert, Dr., Max-Beckmann-Strasse 17, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 003 343 B1

ACTORUM AG.

# Verfahren zur Herstellung von Glyoxal

Die Erfindung betrifft ein Verfahren zur Herstellung von Glyoxal durch Oxidation von Glykol in Gegenwart eines Silberkatalysators bestimmter Korngrösse und unter bestimmten Bedingungen der Temperatur, Verweilzeit und Inertgaskonzentration.

Es ist aus Ullmanns Encyklopädie der technischen Chemie, Band 8, Seiten 250 bis 252, bekannt, dass eines der wichtigsten Verfahren zur Herstellung von Glyoxal die Oxidation von Äthylenglykol mit Luft ist. Als Bedingungen werden Temperaturen von 573 bis 598 K, Kupferoxid als Katalysator und der Zusatz von Halogenverbindungen genannt. Man erhält aus dem Reaktionsgemisch durch Absorption in einer Glyoxallösung oder in Wasser eine 32prozentige Glyoxallösung. Ein solches Verfahren liefert Ausbeuten von höchstens 65 Prozent und Raum-Zeit-Ausbeuten von nur 0,04 bis 1,5 Gramm Glyoxal/$cm^3$ Katalysatorvolumen und Stunde.

Es ist aus Proceed. Acad. Sci. USSR, Chem. Ser. (1964), Seiten 641 bis 643 bekannt, Silber als Katalysator auf Bimsstein und Aluminium als Träger zu verwenden. Es wird festgestellt, dass mit solchen Katalysatoren nur schlechte Ergebnisse erzielt werden. Daher verwendet man Silberspiralen als Katalysator. Das beste Ergebnis wurde mit Silberspiralen, einer Temperatur von 873 K, einem Druck von 544 bis 816 mbar und einer Ausbeute von 69 Prozent erhalten. Der Endstoff fällt in Gestalt einer 25gewichtsprozentigen, wässrigen Lösung mit zusätzlich 5 bis 10 Prozent Glykol an. Aus den mitgeteilten Daten ergibt sich eine Verweilzeit von 0,037 Sekunden oder eine Raum-Zeit-Ausbeute von 4,46 Gramm Glyoxal pro Stunde und $cm^3$ Katalysatorvolumen. Zusätzliches Inertgas wird nicht verwendet. Die Arbeitsweise mit Unterdruck ist auch im Hinblick auf die Raum-Zeit-Ausbeute unbefriedigend.

Die russische Patentschrift 136352 beschreibt die Oxidation von Glykol bei 773 bis 973 K, wobei als Katalysator Silber auf Aluminiumoxid (40% Ag) verwendet wird. Der Katalysator wird vor der Verwendung bei 873 bis 973 K während 2 Stunden geglüht. Die Strömungsgeschwindigkeit beträgt 2,1 Meter pro Sekunde. Die Ausgangsmischung enthält 40 Prozent Glykol und 60 Prozent Wasser. Die Ausbeute beträgt 61 Prozent, die Raum-Zeit-Ausbeute 12,8 Gramm Glyoxal pro Stunde und Gramm Katalysator. Das Verfahren hat den Nachteil, dass der Katalysator umständlich herzustellen ist und eine ziemlich verdünnte Glykollösung eingesetzt werden muss. Wenn der Katalysator verbraucht ist, z.B. durch Vergiftung, muss er in zahlreichen chemischen Operationen aufgearbeitet werden.

In der deutschen Auslegeschrift 1032732 wird dargelegt, dass man bei Verwendung von Kupfer und Silber als Katalysator einen Promotor, z.B. $TiO_2$ und $Mo_2O_5$, benötigt und zur Erhöhung der Ausbeute Hemmstoffe, z.B. HCl, $Cl_2$ oder Äthylenchlorid zusetzen muss. Als bestes Ergebnis erhält man danach eine Raum-Zeit-Ausbeute von 0,043 Gramm pro $cm^3$ und Stunde. Man kann nach der Lehre der Auslegeschrift das Ergebnis verbessern, wenn man das Silber auf Bimsstein, Silicagel oder Aluminiumoxid als Träger aufbringt. Das Verfahren wird zwischen 573 und 723 K unter Verwendung eines Luft-Stickstoffgemisches mit einem Sauerstoffgehalt zwischen 1,6 und 5 Prozent durchgeführt. Bei einer Ausbeute von 55 Prozent wird eine Raum-Zeit-Ausbeute von 0,104 Gramm Glyoxal pro $cm^3$ Katalysatorraum und Stunde erreicht. Die Raum-Zeit-Ausbeute ist unbefriedigend.

Die deutsche Offenlegungsschrift 1923048 beschreibt die Herstellung von Glyoxal und verwendet als Katalysator 2 Komponenten (a und b), und zwar

a) Kupfer oder Silber und/oder Gold und ausserdem

b) Germanium, Zinn, Blei, Stickstoff, Phosphor, Arsen, Antimon und/oder Wismut.

Bevorzugt ist Silber in Verbindung mit Zinn, Phosphor und/oder Arsen und insgesamt insbesondere Kupfer gegenüber Silber. Eine Reaktionstemperatur von ca. 450 bis 880 K, vorzugsweise von ca. 570 bis 720 K, wird angegeben. Verdünnungsgase mit vorzugsweise einem Molverhältnis von Verdünnungsgas zu Sauerstoff im Bereich von 5 bis 200 zu 1 können verwendet werden. Geeignete Verweilzeiten liegen zwischen 0,1 und 20 Sekunden, wobei solche von 1 bis 5 Sekunden bevorzugt sind. Das vergleichsweise zu zahlreichen Beispielen mit Kupferkatalysatoren einzige Beispiel mit einem Kupferfreien Silberkatalysator (Silber/Phosphor) wird bei einer Temperatur von ca. 700 bis 720 K durchgeführt. Aus den Angaben errechnet sich eine unbefriedigende Raum-Zeit-Ausbeute. Nachteilig ist ferner die umständliche Katalysatorherstellung.

In der deutschen Offenlegungsschrift 2634439 wird ein Katalysator verwendet, der aus Phosphor kombiniert mit Cu und/oder Ag besteht. Der Reaktion wird eine Bromverbindung zugesetzt, die dazu ausreicht, die Glyoxalausbeute zu erhöhen, die jedoch nicht so gross ist, dass die Glykoaldehydbildung merklich gesteigert wird oder die Umwandlung des Äthylenglykols auf weniger als etwa 90 Prozent absinkt. Inertgas wird zugesetzt. In den Beispielen wird stets ein Kupfer/Silber/Phosphor-Katalysator verwendet. Die Raum-Zeit-Ausbeute beträgt nur 1,5 Gramm Glyoxal pro $cm^3$ Katalysator und Stunde. Ein weiterer Nachteil ist, dass der Katalysator nur umständlich regeneriert werden kann.

Alle diese Verfahren sind im Hinblick auf einfachen und wirtschaftlichen Betrieb, einfache Katalysatorherstellung und gute Raum-Zeit-Ausbeute unbefriedigend.

Es wurde nun gefunden, dass man kontinuierlich Glyoxal durch Oxidation von Glykol in Gegenwart eines Silberkatalysators bei erhöhter Temperatur vorteilhaft erhält, wenn man in Gegenwart von Silberkristallen mit einer Korngrösse von 0,1 bis 2,5 Millimetern und von Inertgas in einem Verhältnis vom mindestens 4,4 Mol Inertgas je Mol Sauerstoff während einer Verweilzeit von höchstens 0,05 Sekunden bei einer Temperatur von 720 bis 980 K oxidiert.

Die Umsetzung kann durch die folgenden Formeln wiedergegeben werden:

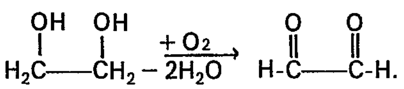

Im Vergleich zu den bekannten Verfahren liefert das Verfahren nach der Erfindung überraschend auf einfacherem und wirtschaftlicherem Wege ein besseres Gesamtergebnis mit Bezug auf Ausbeute, Raum-Zeit-Ausbeute und Reinheit des Endstoffs sowie Lebensdauer des Katalysators. Die Raum-Zeit-Ausbeute ist vergleichsweise besser. Eine umständliche Katalysatorherstellung bzw. Katalysatoraufarbeitung wird vermieden.

Im Hinblick auf die beschriebenen Verfahren besitzt der erfindungsgemässe Katalysator eine höhere Lebensdauer und kann auf einfacherem und wirtschaftlicherem Wege erhalten werden. Silberkristalle aller Teilchengrösse, wie sie auch bei der elektrolytischen Herstellung des Silbergranulats anfallen, können verwendet werden. Bei dem erfindungsgemässen Verfahren werden somit die Elektrolysieranlagen besser ausgenutzt und können entsprechend dimensioniert werden; Energie, Betriebspersonal und Hilfsstoffe, z.B. Salpetersäure, werden eingespart und Operationen wie Wäsche, Siebung und Trocknung des Silbers vereinfacht. Bei den bekannten Verfahren muss man das Silber auf den Träger erst noch aufbringen oder das Silber nachträglich dosieren. Im Falle der Silberspiralen müssen diese erst handwerklich hergestellt werden. Alle diese vorteilhaften Ergebnisse des erfindungsgemässen Verfahrens sind überraschend. Man konnte im Hinblick auf den Stand der Technik nicht vermuten, dass durch Verwendung von reinen Silberkristallen besonderer Korngrösse anstatt Silberspiralen oder Silber auf Trägern oder Silber mit Promotoren (Phosphor) eine Steigerung der Reaktionsgeschwindigkeit und damit der Raum-Zeit-Ausbeute, und noch dazu in wesentlichem Masse, möglich ist.

Für das Verfahren geeignete Ausgangsstoffe sind reines Äthylenglykol, technisches Äthylenglykol bzw. Rohäthylenglykol oder vorteilhaft deren Mischungen mit Wasser; die Konzentration der wässrigen Gemische kann zweckmässig zwischen 30 und 70 Gewichtsprozent, vorzugsweise zwischen 45 und 55 Gewichtsprozent Äthylenglykol schwanken. Vorteilhaft verwendet man 49,5- bis 50,5gewichtsprozentige Lösungen bzw. Mischungen der beiden Komponenten in gleichen Gewichtsanteilen. Das Äthylenglykol wird in Dampfform, vorteilhaft im Gemisch mit Wasserdampf und gegebenenfalls mit Inertgas, dem Reaktionsraum zugeführt. Als unter den Reaktionsbedingungen inerte Gase (Inertgas) werden zweckmässig Edelgase wie Xenon, Argon, Neon, Helium; Alkane wie Methan, Äthan, Propan, 2,2-Dimethylpropan, Butan, Pentan, Isoburan; gasförmige organische Verbindungen anorganischer Elemente wie Tetramethylsilan; Äther wie Dimethyläther, Methyläthyläther; bevorzugt Stickstoff, Kohlenmonoxid und/oder Kohlendioxid; und entsprechende Gemische verwendet. Das Inertgas kann für sich allein oder im Gemisch mit Wasserdampf und/oder Glykoldampf oder vorteilhaft im Gemisch mit Luft verwendet werden. Das Molverhältnis von Inertgas zu Sauerstoff beträgt mindestens 4,4, zweckmässig 4,4 bis 20, vorteilhaft 6 bis 10 zu 1. Die Angaben bezüglich Inertgas beziehen sich hier stets auf die Gesamtmenge, d.h. einschliesslich des Inertgasanteils der bevorzugt verwendeten Luft. Gegebenenfalls kann auch das Abgas der Reaktion selbst als Inertgas verwendet werden, da es in der Regel neben den Inertgasen Stickstoff, Kohlenmonoxid, Kohlendioxid, Wasserstoff und Wasserdampf nur noch Reste unumgesetzten Ausgangsstoffs enthält, der so wiederverwertet wird. Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Äthylenglykol werden zweckmässig im Molverhältnis von 0,5 bis 1,2, insbesondere von 0,7 bis 1 Mol Sauerstoff je Mol Äthylenglykol angewandt. Vorzugsweise beträgt die Gesamtmenge an Wasserdampf nicht mehr als 5, vorteilhaft von 1 bis 4 Mol je Mol Äthylenglykol. Die Luft und gegebenenfalls das Inertgas können direkt in die Verdampfung des Äthylenglykols, zweckmässig in die siedende Äthylenglykol/Wasser-Mischung, oder an einer beliebigen Stelle vor dem Katalysator eingeleitet werden. Die Verweilzeit im Reaktionsraum beträgt höchstens 0,05, vorteilhaft von 0,0005 bis 0,04, zweckmässig von 0,001 bis 0,03, bevorzugt 0,001 bis 0,021 Sekunden.

Die Gesamtschichtdicke des Katalysators beträgt zweckmässig 15 bis 50, vorzugsweise 20 bis 30 Millimeter. Die Katalysatorteilchen in Gestalt von Silberkristallen befinden sich vorteilhaft im Katalysator des üblicherweise vertikal aufgestellten Reaktors in einer Schicht oder je nach Korngrösse in einem oberen oder unteren Teil der Gesamtschicht oder je nach Korngrösse in einem oberen, mittleren oder unteren Teil der Gesamtschicht angeordnet. Das gesamte Katalysatorbett liegt zweckmässig auf einem Netz aus Silber oder Edelstahl (vorgeglüht). Bei grossen Reaktoren mit einem Durchmesser von mehr als 15 cm wird das Netz zweckmässig vor Einbau gewellt. Das Netzt liegt vorteilhaft auf einem Lochboden. Unmittelbar unter dem Lochboden befindet sich zweckmässig ein Wasserkühler. Das Ausgangsgemisch aus Äthylenglykoldampf, Inertgas und Sauerstoff bzw. Luft und gegebenenfalls Wasserdampf wird im allgemeinen von oben nach unten geführt, so dass die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäss alle Angaben der Beschreibung über oberen (unteren) Teil des Katalysators für den entsprechenden, dem Ausgangsgemisch (dem abgeführten Reaktionsgemisch) zugewandten Teil, z.B. horizontal angeordneten Reaktoren für den vorderen (hinteren) Teil des Katalysators. Hat der Katalysator nur eine Schicht, so enthält diese Silberkristalle mit einer Korngrösse von 0,1 bis 2,5, vorteilhaft 0,1 bis 2, vorzugsweise 0,2 bis 1 Millimeter. Bei einem 2-Schichten-Katalysator enthält zweckmässig die obere Teilschicht 20 bis 60, vorzugsweise von 25 bis 50 Gewichtsprozent des Katalysators mit Teilchen der Korngrösse 0,1 bis 0,75 Millimeter, die untere Teilschicht 40 bis 80, vorzugsweise von 50 bis 75 Gewichtsprozent des Katalysators mit Teilchen der Korngrösse 0,75 bis 2,5, vorteilhaft von 0,75 bis 1 Millimeter.

Bei einem 3-Schichten-Katalysator befinden sich im unteren Teil zweckmässig 72,5 bis 89, vorzugsweise 77,5 bis 82,5 Gewichtsprozent aller Katalysatorteilchen, im mittleren Teil 2,5 bis 7,5, vorzugsweise 4,5 bis 6,5 Gewichtsprozent aller Katalysatorteilchen, im oberen Teil 8,5 bis 20, vorzugsweise 13 bis 16 Gewichtsprozent aller Katalysatorteilchen. Die Teilchen des unteren Schichtteils haben Korngrössen von 1 bis 2,5, die des mittleren Schichtteils von 0,75 bis 1, die des oberen Schichtteils 0,1 bis 0,75 Millimeter.

Zweckmässig belastet man den Katalysator mit 0,2 bis 3 t, insbesondere 0,3 bis 1 t Äthylenglykol je $m^2$ Katalysatorbettquerschnitt und Stunde. Zur grosstechnischen Ausführung verwendet man bevorzugt Katalysatorbettdurchmesser von mindestens 0,2, zweckmässig 0,5 bis 2 Meter.

Für die Oxidation leitet man ein Gasgemisch aus Äthylenglykoldampf, Luft, Inertgas und zweckmässig Wasserdampf in vorgenannten Mengen bei Temperaturen von 720 bis 980 K, vorzugsweise von 770 bis 980 K, durch den Silberkatalysator. Das Verfahren wird im allgemeinen bei Drücken zwischen 0,8 und 2 bar, vorzugsweise zwischen 0,8 und 1,8 bar, insbesondere 1,05 bis 1,5 bar, kontinuierlich durchgeführt. Es ist dabei vorteilhaft, die die Katalysatorzone verlassenden Reaktionsgase innerhalb kurzer Zeit abzukühlen, z.B. auf Temperaturen von 270 bis 370 K. Das abgekühlte Gasgemisch wird dann zweckmässig einem Absorptionsturm zugeführt, in welchem der Endstoff mit Wasser oder vorteilhafter einer 20- bis 50gewichtsprozentigen, wässrigen Glyoxallösung, bevorzugt im Gegenstrom, aus dem Gasgemisch gewaschen wird. Im allgemeinen werden die wässrigen Lösungen, zweckmässig in 40gewichtsprozentiger Form, direkt weiterverwendet, gegebenenfalls kann man den Endstoff in üblicher Weise, z.B. durch azeotrope Destillation mit Äthylbenzol und fraktionierter Destillation des Rückstands, isolieren.

Gegebenenfalls kann man auch Hemmstoffe dem Ausgangsgemisch, zweckmässig in den Dampf/Gas-Strom der übrigen Komponeneten vor dem Katalysator, zugeben. Vorteilhaft sind Halogenkohlenwasserstoffe, insbesondere Brom- und Chlorkohlenwasserstoffe, z.B. Tetrachloräthylen, 1,1,2,2- oder 1,1,1,2-Tetrachloräthan, Amylchlorid, Cyclohexylchlorid, 1,2-Dichlorpropan, Methylenchlorid, Dichlorbutan, Isopropylbromid, n-Propylbromid, Butylbromid, Chloroform, Bromoform, Äthyljodid, Propyljodid, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1,-Dichloräthan, n-Propylchlorid, 1,2-cis-Dichloräthylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; und entsprechende Gemische. Bevorzugte Hemmstoffe sind insbesondere HCl, $Cl_2$, Bromoform. Zweckmässig verwendet man den Hemmstoff in einer Menge von 0,01 bis 0,8 Gewichtsprozent, vorzugsweise von 0,05 bis 0,5 Gewichtsprozent, bezogen auf Äthylenglykol.

Das nach dem Verfahren der Erfindung herstellbare Glyoxal ist Knitterfestmittel, Hilfsmittel zur Erhöhung der Reissfestigkeit und Elastizität von Faserstoffen, Gerbstoff, Härter in der photographischen Industrie und wertvoller Ausgangsstoff für die Herstellung von Kunstharzen, Textilhilfsmitteln, z.B. zur Verhinderung des Einlaufens nach dem Waschen, Papierhilfsmitteln, z.B. zur Erhöhung der Nassfestigkeit, und Kunststoffen. Bezüglich der Verwendung wird auf vorgenannte Veröffentlichungen und Ullmann (loc. cit.) verwiesen.

Die in den folgenden Beispielen angeführten Teile bedeuten Gewichtsteile.

Beispiel 1

Man verwendet eine Anlage mit Äthylenglykol-Verdampfer und einem senkrechten Rohrreaktor. Der Reaktor enthält an seinem Kopf die Zuführung für das dampfförmige Ausgangsgemisch und die Reaktorhaube. Die Katalysatorschicht liegt unterhalb des Reaktorkopfes, weiter unten folgt eine Kühlzone. Der Reaktor ist mit 4 Absorptionskolonnen verbunden.

In den Reaktor wird ein Katalysator aus Silberkristallen (45 Teilen) folgender Zusammensetzung eingetragen:

| | Anteil am Katalysator (Gew.%) | Korngrösse mm |
|---|---|---|
| Schicht 1 | 15 | 0,1 -0,75 |
| Schicht 2 | 5 | 0,75-1 |
| Schicht 3 | 80 | 1 -2,5 |

Die Höhe des Katalysators beträgt 30 mm. Dem Verdampfer wird pro Stunde ein Gemisch von 246 Teilen Äthylenglykol, 246 Teilen Wasser und 456 Teilen Luft zugeführt und verdampft. Das dampfförmige Ausgangsgemisch wird, nachdem ihm stündlich 340 Teile Stickstoff als Inertgas zugesetzt werden, durch den Katalysator geleitet und bei 870 K und 1,12 bar umgesetzt. Die Verweilzeit beträgt 0,02 Sekunden. Das Reaktionsgemisch wird nun auf 320 K abgekühlt und in einer 27,5gewichtsprozentigen, wässrigen Glyoxallösung, die 1,2 Prozent Glykol enthält, gelöst. Das zu absorbierende Gemisch hat die gleiche Zusammensetzung, so dass sich die Konzentration nicht ändert. Während der Absorption wird ständig eine der neugebildeten Menge entsprechende Menge Endstoff abgezogen. In Form einer 27,5gewichtsprozentigen Glyoxallösung erhält man 152 Teile pro Stunde Glyoxal, entsprechend einer Ausbeute von 66% der Theorie, bezogen auf verwendetes Glykol. Die Lebensdauer des Katalysators beträgt 90 Tage. Die Glyoxallösung hat einen Gehalt von 1,2 Gewichtsprozent Äthylenglykol und 1,6 Gewichtsprozent Formaldehyd. Der Umsatz beträgt 97,8 Prozent, die Raum-Zeit-Ausbeute 14,6 g Glyoxal pro $cm^3$ Katalysatorvolumen und Stunde.

Beispiel 2

Es wird die gleiche Anlage wie in Beispiel 1 verwendet. Die Umsetzung wird analog Beispiel 1 durchgeführt, wobei stündlich dem dampfförmigen Ausgangsgemisch vor dem Katalysator 0,1 Teile Bromoform zugesetzt werden.

In Form einer 33,0gewichtsprozentigen Glyoxallösung erhält man 153 Teile pro Stunde Glyoxal, entsprechend einer Ausbeute von 66,4% der Theorie. Die Lebensdauer des Katalysators beträgt 85 Tage. Die Glyoxallösung hat einen Gehalt von 0,4 Gewichtsprozent Glykol und 1,5 Gewichtsprozent Formaldehyd. Der Umsatz beträgt 98 Prozent, die Raum-Zeit-Ausbeute 14,6 Gramm Glyoxal pro $cm^3$ Katalysatorvolumen und Stunde.

Beispiel 3

Es wird die gleiche Anlage wie in Beispiel 1 verwendet. In den Reaktor wird ein Katalysator aus Silberkristallen (45 Teilen) folgender Zusammensetzung eingetragen:

| | Anteil am Katalysator (Gew.%) | Korngrösse mm |
|---|---|---|
| Schicht 1 | 30 | 0,1-0,75 |
| Schicht 2 | 70 | 0,75-1 |

Die Umsetzung wird analog Beispiel 1 durchgeführt.

In Form einer 27,5gewichtsprozentigen Glyoxallösung erhält man 152 Teile pro Stunde Glyoxal, entsprechend einer Ausbeute von 66% der Theorie. Die Lebensdauer des Katalysators beträgt 80 Tage. Die Glyoxallösung hat einen Gehalt von 1,2 Gewichtsprozent Glykol und 1,4 Gewichtsprozent Formaldehyd. Der Umsatz beträgt 97,9 Prozent, die Raum-Zeit-Ausbeute 14,6 Gramm Glyoxal pro $cm^3$ Katalysatorvolumen und Stunde.

**Patentanspruch**

Verfahren zur kontinuierlichen Herstellung von Glyoxal durch Oxidation von Glykol in Gegenwart eines Silberkatalysators bei erhöhter Temperatur, dadurch gekenzeichnet, dass man in Gegenwart von Silberkristallen mit einer Korngrösse von 0,1 bis 2,5 Millimeter und von Inertgas in einem Verhältnis von mindestens 4,4 Mol Inertgas je Mol Sauerstoff während einer Verweilzeit von höchstens 0,05 Sekunden bei einer Temperatur von 720 bis 980 K oxidiert.

**Claim**

A process for the continuous preparation of glyoxal by oxidizing glycol in the presence of a silver catalyst at an elevated temperature, wherein this oxidation is carried out in the presence of silver crystals having a particle size of from 0.1 to 2.5 millimeters and of an inert gas in a ratio of at least 4.4 moles of inert gas per mole of oxygen, with a residence time of at most 0.05 second, at from 720 to 980° K.

**Revendication**

Procédé pour la préparation en continu de glyoxal par oxydation de glycol en présence d'un catalyseur à l'argent à température élevée, caractérisé en ce qu'on oxyde en présence de cristaux d'argent ayant une grosseur de grains de 0,1 à 2,5 mm et en présence de gaz inerte dans un rapport d'au moins 4,4 mol de gaz inerte par mol d'oxygène, pendant une durée de séjour de 0,05 s au maximum, à une température de 720 à 980° K.